(19) Europäisches Patentamt
European Patent Office
Office européen des brevets

(11) Veröffentlichungsnummer : **0 137 351**
**B1**

(12) # EUROPÄISCHE PATENTSCHRIFT

(45) Veröffentlichungstag der Patentschrift : **04.03.87**

(51) Int. Cl.⁴ : **C 07 C 99/00**, C 07 D233/38, C 07 C119/08

(21) Anmeldenummer : **84111024.0**

(22) Anmeldetag : **15.09.84**

(54) **Verfahren zur enantioselektiven Herstellung von alpha-alkylierten, acyclischen alpha-Aminocarbonsäuren.**

(30) Priorität : **27.09.83 DE 3334855**

(43) Veröffentlichungstag der Anmeldung : **17.04.85 Patentblatt 85/16**

(45) Bekanntmachung des Hinweises auf die Patenterteilung : **04.03.87 Patentblatt 87/10**

(84) Benannte Vertragsstaaten : **AT BE CH DE FR GB IT LI LU NL SE**

(56) Entgegenhaltungen :
**SYNTHESIS, INTERNATIONAL JOURNAL OF METHODS IN SYNTHETIC ORGANIC CHEMISTRY, Nr. 3, März 1984, Seiten 271-274; U. SCHÖLLKOPF u.a.: "Asymmetric syntheses via heterocyclic intermediates; XXI. Enantioselective synthesis of alpha-alkylleucine methyl esters using the bis-lactim ether of cyclo(L-Leu-L-Leu) as starting material"**
**CHEMICAL ABSTRACTS, Band 101, Nr. 21, 19. November 1984, Seite 755, Nr. 191762m, Columbus, Ohio, US; D. SEEBACH u.a.: "Alpha-alkylation of alpha-heterosubstituted carboxylic acids without racemization. EPC-syntheses of tertiary alcohols and thiols"**

(73) Patentinhaber : **Degussa Aktiengesellschaft**
**Weissfrauenstrasse 9**
**D-6000 Frankfurt am Main 1 (DE)**

(72) Erfinder : **Seebach, Dieter, Prof., Dr.**
**Orellistrasse 3**
**CH-8044 Zürich (CH)**
Erfinder : **Naef, Reto, Dipl.-Chem.**
**Schärenmoostrasse 38**
**CH-8052 Zürich (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 137 351 B1

Jouve, 18, rue St-Denis, 75001 Paris, France

**Beschreibung**

Die Erfindung betrifft ein neues Verfahren zur enantioselektiven Herstellung von α-alkylierten, acyclischen α-Aminocarbonsäuren der allgemeinen Formel

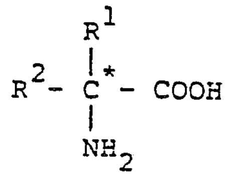

(I)

in der * ein Asymmetrizentrum, $R^1$ eine Methyl-, Ethyl-, n-Propyl-, n-Butyl-, Allyl-, Benzyl- oder substituierte Benzylgruppe und $R^2$ eine Methyl-, Ethyl-, n-Propyl-, i-Propyl-, n-Butyl-, sekundäre Butyl-, i-Butyl-, Methoxymethyl-, Methylmercaptomethyl-, 2-Methylmercaptoethyl-, 2-Ethylmercaptoethyl-, Phenyl-, Benzyl- oder eine in beliebiger Ringstellung 1- bis 3-fach durch eine Alkyl- oder Alkoxygruppe oder durch Fluor oder Chlor substituierte Benzylgruppe bedeuten, welches dadurch gekennzeichnet ist, daß man ein Imidazolidin-4-on der allgemeinen Formel

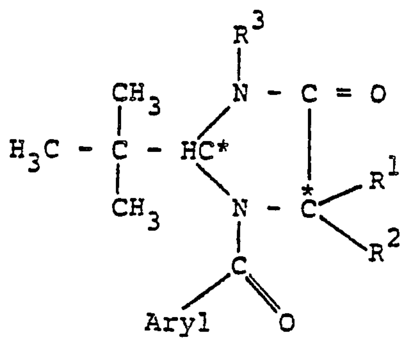

(II)

in der *, $R^1$ und $R^2$ die bereits angegebene Bedeutung haben und $R^3$ eine Methyl- oder Ethylgruppe und Aryl eine Phenyl- oder substituierte Phenylgruppe bedeuten, sauer verseift.

Das erfindungsgemäße Verfahren geht letztlich von enantiomerenreinen acyclischen α-Aminocarbonsäuren aus, erfordert keinen zusätzlichen chiralen Hilfsstoff, beispielsweise eine zweite optisch aktive α-Aminocarbonsäure oder optisch aktives Phenylethylamin und ermöglicht es, aus einem Edukt-Enantiomeren, d. h. aus einer (S)- oder aus einer (R)-α-Aminocarbonsäure, wahlweise die beiden enantiomeren Formen der gewünschten α-Alkyl-α-aminocarbonsäure herzustellen.

Zur Durchführung des erfindungsgemäßen Verfahrens wird zunächst eine (S)- oder (R)-α-Aminocarbonsäure der allgemeinen Formel

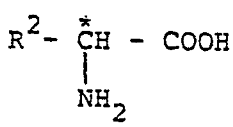

(IX)

in der * ein Asymmetriezentrum und $R^2$ eine Methyl-, Ethyl-, n-Propyl-, i-Propyl-, n-Butyl-, sekundäre Butyl-, i-Butyl-, Methoxymethyl-, Methylmercaptomethyl-, 2-Methylmercaptoethyl-, 2-Ethylmercaptoethyl-, Phenyl-, Benzyl- oder eine in beliebiger Ringstellung 1- bis 3-fach durch eine Alkyl- oder Alkoxygruppe oder durch Fluor oder Chlor substituierte Benzylgruppe bedeuten, in an sich bekannter Weise durch Umsetzung eines ihrer Ester mit einem niedrigen Alkanol, insbesondere Methanol oder Ethanol, mit Monoethylamin oder vorzugsweise Monomethylamin in das entsprechende α-Aminocarbonsäureamid der allgemeinen Formel

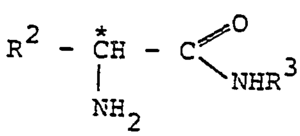

(VIII)

in der * und $R^2$ die bereits angegebene Bedeutung haben und $R^3$ eine Methyl- oder Ethylgruppe bedeutet, umgewandelt.

Als einzusetzende (S)- bzw. (R)-α-Aminocarbonsäuren der allgemeinen Formel (IX) kommen

beispielsweise in Frage Alanin, Phenylalanin, substituierte Phenylalanine, wie 3,4-Dimethoxyphenylalanin, Phenylglycin, substituierte Phenylglycine, wie 4-Methoxyphenylglycin, 2-Aminobuttersäure, Valin, Norvalin, Leucin, Isoleucin, Norleucin, Methionin, S-Methylcystein oder O-Methylserin.

Das α-Aminocarbonsäureamid der allgemeinen Formel (VIII) wird dann durch Kondensation mit Pivalaldehyd in ein Imin (Schiff'sche Base) der allgemeinen Formel

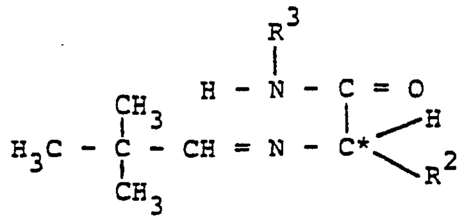

(VII)

in der *, $R^2$ und $R^3$ die bereits angegebene Bedeutung haben, überführt. Die Reaktion wird zweckmäßigerweise so durchgeführt, daß das chirale α-Aminocarbonsäureamid der allgemeinen Formel (VIII) zusammen mit Pivalaldehyd in einem inerten Lösungsmittel bis zur vollständigen Wasserabscheidung an einem Wasserabscheider gekocht wird. Geeignete inerte Lösungsmittel sind beispielsweise aliphatische oder aromatische Kohlenwasserstoffe, wie n-Pentan, n-Hexan, Cyclohexan, Benzol oder Toluol und Halogenkohlenwasserstoffe, wie Methylenchlorid, Chloroform oder 1,2-Dichlorethan. Nach Abtrennung des abgeschiedenen Wassers und des restlichen Lösungsmittels fallen die Imine der allgemeinen Formel (VII) in sehr reiner Form an und können ohne weitere Reinigung direkt weiter umgesetzt werden.

Bei dieser weiteren Umsetzung werden sie unter gleichzeitiger oder anschließender Einführung der Gruppe

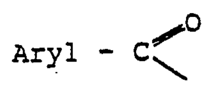

in der Aryl eine Phenyl- oder substituierte Phenylgruppe bedeutet, cyclisiert. Je nach den angewandten Reaktionsbedingungen erhält man bei dieser Cyclisierungsreaktion zwei unterschiedliche Diastereomere, da ein zweites Asymmetriezentrum neu gebildet wird. Setzt man das Imin der allgemeinen Formel (VII) bei einer Temperatur zwischen 110 und 150 °C, vorzugsweise etwa 130 °C, in Abwesenheit eines Lösungsmittels mit Benzoesäureanhydrid oder einem substituierten Benzoesäureanhydrid um, so erhält man das entsprechende Imidazolidin-4-on der allgemeinen Formel

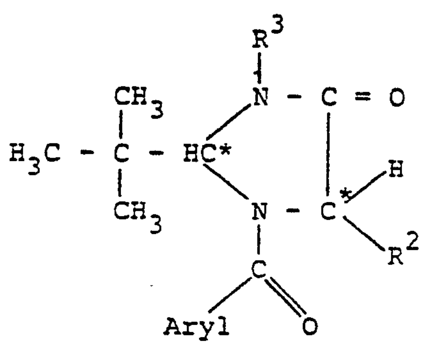

(V)

in der *, $R^2$, $R^3$ und Aryl die bereits angegebene Bedeutung haben, in der cis-Form, bei der die tert. Butylgruppe und der Substituent $R^2$ cis-ständig sind. Das molare Verhältnis des eingesetzten Benzoesäureanhydrids zu dem Imin der allgemeinen Formel (VII) soll zweckmäßigerweise 1,0 bis 1,2 : 1, vorzugsweise 1,1 : 1, betragen.

Wird dagegen das Imin der allgemeinen Formel (VII) bei relativ tiefer Temperatur zuerst mit methanolischer Salzsäure und dann mit einem Benzoesäurehalogenid oder substituierten Benzoesäurehalogenid in Gegenwart eines als Säureakzeptor dienenden tertiären Amins, wie Triethylamin oder Pyridin, umgesetzt, so entsteht das entsprechende Imidazolidin-4-on der allgemeinen Formel (V) in der trans-Form, bei der die tert. Butylgruppe und der Substituent $R^2$ trans-ständig sind. Die zweckmäßigen Temperaturen für die Umsetzung mit der methanolischen Salzsäure liegen zwischen — 10 und + 40 °C, vorzugsweise zwischen 0 und + 25 °C. Die methanolische Salzsäure wird im Überschuß (mindestens 5

molar, bezogen auf das eingesetzte Imin) angewandt und die Umsetzung erfordert eine Zeit zwischen etwa 2 und 3 Stunden. Dann wird das $HCl/CH_3OH$-Gemisch entfernt und der Rückstand in Methylenchlorid aufgenommer und mit einem Äquivalent des Benzoesäurehalogenids und 2 Äquivalenten eines tertiären Amins versetzt. Die zweckmäßigsten Temperaturen für diese Umsetzung liegen ebenfall wieder zwischen 0 und + 25 °C. Das Reaktionsgemisch wird mit Sodalösung neutralisiert, die organische Phase wird unter vermindertem Druck eingeengt und der Rückstand im Vakuum bei einer Temperatur zwischen 30 und 60 °C getrocknet.

Bei der Umwandlung der Imine der allgemeinen Formel (VII) in die entsprechenden Imidazolidin-4-one der allgemeinen Formel (V) werden als Benzoesäureanhydrid bzw. Benzoesäurehalogenid vorzugsweise das unsubstituierte Benzoesäureanhydrid bzw. Benzoylchlorid verwendet. Es lassen sich aber selbstverständlich auch kernsubstituierte Benzoesäureanhydride bzw. Benzoesäurehalogenide einsetzen.

Die erhaltenen rohen cis- bzw. trans-Imidazolidin-4-one werden, falls notwendig durch Umkristallisation weiter gereinigt.

Das Imidazolidin-4-on der allgemeinen Formel (V) in der cis- oder trans-Form wird anschließend mit einer starken Base der allgemeinen Formel

M—Y (VI),

in der M für Lithium, Natrium oder Kalium steht und Y Wasserstoff oder eine n-Butyl-, tert. Butylat-, Amino-, Dimethylamino-, Diethylamino-, Di-n-propylamino, Di-iso-propylamino-, Di-(trimethylsilyl)-amino- oder Phenylgruppe bedeutet, umgesetzt. Diese Umsetzung wird zweckmäßigerweise so durchgeführt, daß man die starke Base der allgemeinen Formel (VI) in einer Menge von 1,0 bis 1,1 Äquivalenten auf einmal oder innerhalb weniger Minuten zu einer Lösung des Imidazolidin-4-ons in einem inerten Lösungsmittel zugibt. Geeignete inerte Lösungsmittel sind beispielsweise Ether, wie Di-ethylether, Di-n-propylether, Methyl-tert.-butylether oder Tetrahydrofuran, und auch Kohlenwasserstoffe, wie n-Pentan, n-Hexan oder Cyclohexan. Gegebenenfalls können auch Gemische aus solchen Ethern und Kohlenwasserstoffen eingesetzt werden.

Als starke Basen der allgemeinen Formel (VI) können beispielsweise Butyllithium, Phenyllithium, Natriumhydrid, Kalium-tert. butylat oder N,N-disubstituierte Lithiumamide Verwendung finden. Bevorzugt werden Bytyllithium und Lithiumdiisopropylamid. Die zweckmäßigsten Reaktionstemperaturen liegen zwischen —80 und 0 °C.

Bei dieser Umsetzung werden Enolate der allgemeinen Formel

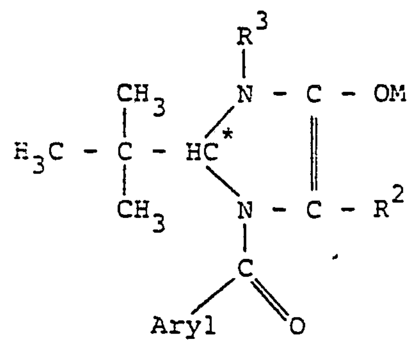

(III)

gebildet, in der *, $R^2$, $R^3$, Aryl und M die bereits angegebene Bedeutung haben.

Diese in Lösung vorliegenden Enolate werden anschließend, zweckmäßigerweise ebenfalls wieder bei einer Temperatur zwischen —80 und 0 °C, mit einem Alkylierungsmittel der allgemeinen Formel

$R^1$—X (IV)

weiter umgesetzt, in der $R^1$ eine Methyl-, Ethyl-, n-Propyl-, n-Butyl-, Allyl-, Benzyl- oder substituierte Benzylgruppe und X eine Austrittsgruppe aus der Reihe Chlorid, Bromid, Jodid, Tosylat, Mesylat oder Trifluormethylsulfonat bedeuten.

Wenn $R^1$ eine substituierte Benzylgruppe bedeutet, kann diese in beliebiger Ringstellung 1- bis 3-fach durch Fluor, Chlor, Brom oder eine Methoxy-, Ethoxy-, Methyl-, Ethyl- oder Isopropylgruppe substituiert sein.

Das Alkylierungsmittel der allgemeinen Formel (IV) wird zweckmäßigerweise in einem 1,1- bis 1,2-fachen molaren Überschuß, bezogen auf das ursprünglich zur Enolatbildung eingesetzte Imidazolidin-4-on der allgemeinen Formel (V) angewandt.

Bei der Alkylierungsreaktion entsteht ein Imidazolidin-4-on der allgemeinen Formel .

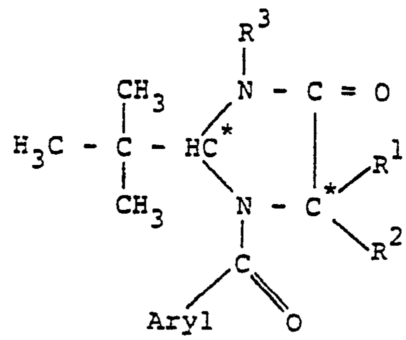

(II)

in der *, $R^1$, $R^2$, $R^3$ und Aryl die bereits angegebene Bedeutung haben.

Der stereochemische Verlauf der Alkylierungsreaktion ist so, daß die tert. Butylgruppe und der Substituent $R^2$ in dem Imidazolidin-4-on der allgemeinen Formel (II) immer cis-ständig und die tert. Butylgruppe und der neu eingeführte Substituent $R^1$ immer trans-ständig sind. Infolge der unterschiedlichen Konfiguration der die tert. Butylgruppe und den Substituenten $R^2$ tragenden Kohlenstoffatome in den Imidazolidin-4-onen der allgemeinen Formel (V) lassen sich somit letztlich, ausgehend von einem Enantiomeren einer α-Aminocarbonsäure, beide enantiomeren Formen der herzustellenden α-Alkyl-α-aminocarbonsäure herstellen.

Die Imidazolidin-4-one der allgemeinen Formel (II) werden schließlich sauer zu den α-alkylierten, acyclischen α-Aminocarbonsäuren der allgemeinen Formel

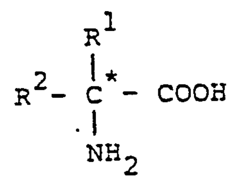

(I)

verseift, in der *, $R^1$ und $R^2$ die bereits angegebene Bedeutung haben. Dies geschieht zweckmäßigerweise durch Erhitzen mit einer relativ konzentrierten Mineralsäure auf eine Temperatur zwischen 150 und 200 °C unter Druck. Als Säuren werden vor allem 20 bis 36 gewichtsprozentige Salzsäure oder 30 bis 48 gewichtsprozentige wäßrige Bromwasserstoffsäure eingesetzt. Es kann aber auch 10 bis 50 gewichtsprozentige wäßrige Schwefelsäure verwendet werden.

Das Hydrolysegemisch wird abgekühlt, filtriert, mit Methylenchlorid extrahiert und die wäßrige Phase im Vakuum eingedampft. Der Rückstand wird in Wasser aufgenommen und in an sich bekannter Weise mit Hilfe eines Ionenaustauschers oder von Propylenoxid von der enthaltenen Mineralsäure befreit. Die verbleibende wäßrige Lösung der α-alkylierten, acyclischen α-Aminocarbonsäure der allgemeinen Formel (I) wird eingedampft und unter vermindertem Druck bis zur Gewichtskonstanz getrocknet. Man erhält so in guten chemischen Ausbeuten und in optischen Ausbeuten von 90 bis 95 % die kristallinen Säuren.

Das erfindungsgemäße Verfahren eröffnet somit einen hervorragenden Zugang zu den α-alkylierten, acyclischen α-Aminocarbonsäuren der allgemeinen Formel (I). Beispielsweise kann auf diesem Wege (—)-α-Methyldopa, ein wichtiges Pharmazeutikum, aus (S)-Alanin auf einfache Weise hergestellt werden. Die Chiralität der Ausgangsaminocarbonsäure reproduziert sich selbst. Es ist daher für die gesamte Reaktionsfolge kein zusätzliches optisch aktives Hilfsreagenz erforderlich.

Das erfindungsgemäße Verfahren wird durch die nachfolgenden Beispiele näher erläutert :

## Beispiel 1

a) Herstellung von (S)-N-(2′, 2′-Dimethylpropyliden)-valinmonoethylamid

Zu 112,0 g (864 mmol) (S)-Valinmonomethylamid, gelöst in 300 ml n-Pentan, wurden 95,7 ml (870 mmol) Pivalaldehyd zugegeben. Das Reaktionsgemisch wurde am Wasserabscheider gekocht, bis die Wasserbildung beendet war (4 Stunden). Das Lösungsmittel wurde unter vermindertem Druck entfernt und es hinterblieben 130 g (76 % der Theorie) an (S)-N-(2′,2′-Dimethylpropyliden)-valinmonomethylamid, das ohne weitere Reinigung weiterverarbeitet wurde. Für die Ermittlung der exakten Stoffdaten wurde eine Probemenge durch Destillation gereinigt.

Siedepunkt : 80 °C/0,013 mbar
Schmelzpunkt : 69 °C
Elementaranalyse :
$C_{11}H_{22}N_2O$ (214,29)
Berechnet : %C 66,62 %H 11,18 %N 14,13

Gefunden : %C 66,78 %H 11,11 %N 14,17
IR ($CDCl_3$) : 3 400 (m), 2 955 (s), 1 665 (s), 1 520 (m), 1 460 (m), 1 365 (m) $cm^{-1}$.
$^1$H-NMR ($CDCl_3$) : 7,46 (s, 1H) CH = N ;
6,43 (š, 1H) NH ;
3,23 (d, J = 5 Hz, 1H) CH—N ;
2,77 (d, J = 6 Hz, 3H) $CH_3$—N ;
2,16 (m, 1H) CH—C ;
1,13 (s, 9H) $(CH_3)_3$—C ;
0,87 ppm (d, J = 6 Hz, 6H) $(CH_3)_2$—C.

b) Herstellung von (2R, 5S)-1-Benzoyl-2-tert.butyl-5-methyl-ethyl-3-methyl-imidazolidin-4-on

52,0 g (260 mmol) des nach a) hergestellten (S)-N-(2',2'-Dimethylpropyliden)-valinmonomethylamids wurden mit 64,6 g (286 mmol) Benzoesäureanhydrid eine Stunde lang auf 140 °C erhitzt. Nach dem Abkühlen wurde die erstarrte Masse in 500 ml Methylenchlorid aufgenommen und die Methylenchloridlösung zweimal mit je 300 ml 2 N Sodalösung und einmal mit 200 ml Wasser ausgewaschen. Die organische Phase wurde über $MgSO_4$ getrocknet und das Methylenchlorid unter vermindertem Druck abdestilliert. Der Rückstand wurde zur Reinigung einmal mit kaltem Diethylether gewaschen. Ausbeute an (2R,5S)-1-Benzoyl-2-tert.butyl-5-(methylethyl)-3-methyl-imidazolidin-4-on : 66,6 g (82 % der Theorie).
Schmelzpunkt : 112 °C
$[\alpha]_D^{25}$ : + 22,4° (c = 1,7 ; $CHCl_3$)
Elementaranalyse :
$C_{18}H_{26}N_2O_2$ (302,39)
Berechnet : %C 71,49 %H 8,67 %N 9,26
Gefunden : %C 71,57 %H 8,75 %N 9,39
IR ($CHCl_3$) : 2 965 (m), 1 685 (s), 1 635 (s), 1 362 (s), 1 295 (m) $cm^{-1}$.
$^1$H-NMR ($CCl_4$) : 7,46-7,13 (m, 5H) $H_{Ar}$ ;
5,33 (s, 1H) CH ;
3,86 (d, J = 10 Hz, 1H) CH—CH $(CH_3)_2$ ;
2,97 (s, 3H) $CH_3$—N ;
2,06-1,46 (m, 1H) CH—CH $(CH_3)_2$ ;
1,03 (s, 9H) $(CH_3)_3$—C ;
1,00 (d, J = 6 Hz, 3H) HC—$CH_3$ ;
0,66 ppm (d, J = 6 Hz, 3H) HC—$CH_3$.

c) Herstellung von (2R, 5S)-1-Benzoyl-2-tert.butyl-3,5-dimethyl-5-(methylethyl)-imidazolidin-4-on

Zu 3,02 g (10,0 mmol) des nach b) hergestellten (2R,5S)-1-Benzoyl-2-tert.butyl-5-(methylethyl)-3-methyl-imidazolidin-4-ons, gelöst in 60 ml Tetrahydrofuran, wurden bei — 78 °C 10,0 mmol einer 1M Lösung von Lithiumdiisopropylamid in Tetrahydrofuran zugegeben. Dabei färbte sich die Lösung tiefrot. Nach weiterem 15-minütigen Rühren bei — 78 °C wurden 15 mmol Methyljodid zugegeben. Das Reaktionsgemisch wurde auf Raumtemperatur erwärmen gelassen. Das jetzt schwach gelbe Reaktionsgemisch wurde in 100 ml etwa halbgesättigter wäßriger $NH_4Cl$-Lösung gegossen und mit insgesamt 200 ml Diethylether extrahiert. Die vereinigten organischen Extrakte wurden mit Wasser gewaschen, über $MgSO_4$ getrocknet und unter vermindertem Druck vom Lösungsmittel befreit. Der Rückstand wurde zur weiteren Reinigung mit einem Gemisch von Diethylether und n-Pentan im Volumenverhältnis 5 : 1 als Laufmittel chromatographiert. Nach dem Abdampfen des Lösungsmittels aus dem Eluat hinterblieben 2,18 g (70 % der Theorie) an (2R,5S)-1-Benzoyl-2-tert.butyl-3,5-dimethyl-5-(methylethyl)-imidazolidin-4-on.
$[\alpha]_D^{25}$ : + 38,7° (c = 1,4 ; $CHCl_3$)
$^1$H—NMR ($CDCl_3$) : 7,43 (s, 5H) $H_{Ar}$ ;
5,46 (s, 1H) C—H ;
3,00 (s, 3H) $CH_3$—N ;
2,26—1,76 (m, 1H) C—CH $(CH_3)_2$ ;
1,37 (s, 3H) C—$CH_3$ ;
1,15 (d, J = 7 Hz, 3H) CH $(CH_3)$ und
1,12 (d, J = 7 Hz, 3H) CH—$(CH_3)$ ;
1,02 ppm (s, 9H) C—$(CH_3)_3$.

d) Herstellung von (S)-α-Methylvalin

1,36 g (4,3 mmol) des nach c) hergestellten (2R,5S)-1-Benzoyl-2-tert.butyl-3,5-dimethyl-5-(methylethyl)-imidazolidin-4-ons wurden mit 30 ml 20 gewichtsprozentiger wäßriger Salzsäure 4 Stunden im Bombenrohr auf 180 °C erhitzt. Das Hydrolysegemisch wurde filtriert, mit 30 ml Methylenchlorid

extrahiert und die wäßrige Phase im Vakuum zur Trockne eingedampft. Der Rückstand wurde in 30 ml Wasser aufgenommen und mittels eines Ionenaustauschers dehydrohalogeniert. Die so erhaltene wäßrige Lösung des freien (S)-α-Methylvalins wurde eingedampft und der kristalline Rückstand bei 50 °C im Vakuum bis zur Gewichtskonstanz getrocknet. Ausbeute 0,535 g (95 % der Theorie).

$[\alpha]_D^{25}$ : — 4,3° (c = 1 ; 0,2N HCl)
$^1$H-NMR ($D_2O$) : 2,56-2,16 (septett, J = 6 Hz, 1H) C<u>H</u> $(CH_3)_2$ ;
1,65 (s, 3H) α—C—C<u>H</u>$_3$ ;
1,12 ppm (d, J = 6 Hz, 6H) CH—(C<u>H</u>$_3$)$_2$ ;
Referenz (HDO) : 4,90 ppm

## Beispiel 2

a) Herstellung von (S)-N-(2',2'-Dimethylpropyliden)-methioninmonomethylamid

Zu 40,0 g (247 mmol) (S)-Methioninmonomethylamid, gelöst in 100 ml n-Pentan, wurden 27,5 ml (250 mmol) Pivalaldehyd zugegeben. Das Reaktionsgemisch wurde am Wasserabscheider gekocht, bis die Wasserbildung beendet war (3 Stunden). Das Lösungsmittel wurde unter vermindertem Druck entfernt und es hinterblieben 52,2 g (92 % der Theorie) an (S)-N-(2',2'-Dimethylpropyliden)-methioninmonomethylamid, das ohne weitere Reinigung weiterverarbeitet wurde. Für die Ermittlung der exakten Stoffdaten wurde eine Probemenge durch Destillation gereinigt.

Siedepunkt : 120 °C/0,13 mbar
$[\alpha]_D^{25}$ : + 10,6° (c = 2,4 ; $CHCl_3$)
IR ($CHCl_3$) : 3 400 (m), 2 960 (s), 1 665 (s), 1 525 (m), 1 365 (m) $cm^{-1}$
MS : 215 ($M^+$—15,1), 173 (100)
$^1$H-NMR (C $Cl_4$) : 7,56 (s, 1H) C<u>H</u> = N ;
6,56 (d, J = 5 Hz, 1H) N<u>H</u> ;
3,66 (dd, $J_1$ = 4 Hz, $J_2$ = 8 Hz, 1H) C—<u>H</u> ;
2,77 (d, J = 5 Hz, 3H) C<u>H</u>$_3$—N ;
2,57-1,56 (m, 4H) (C<u>H</u>$_2$)$_2$ ;
2,02 (s, 3H) C<u>H</u>$_3$—S ;
1,10 ppm (s, 9H) (C<u>H</u>$_3$)$_3$C.

b) Herstellung von (2S, 5S)-1-Benzoyl-2-tert.butyl-3-methyl-5-(3'-thiabutyl)-imidazolidin-4-on

Eine Lösung von 23,0 g (100,0 mmol) des nach a) hergestellten (S)-N-(2',2'-Dimethylpropyliden)-methioninmonomethylamids in 30 ml Methanol wurde unter Kühlung auf 0 °C mit 60 ml einer gesättigten methanolischen Salzsäure versetzt und 30 Minuten bei 0 °C und anschließend 2 Stunden bei 25 °C gerührt. Das Lösungsmittel wurde bei 25 °C unter vermindertem Druck entfernt und der Rückstand in 100 ml Methylenchlorid aufgenommen. Die Methylenchloridlösung wurde bei 0 °C mit 11,6 ml (100 mmol) Benzoylchlorid und 27,7 ml (200 mmol) Triethylamin versetzt. Nach dem Erwärmen auf 25 °C wurde das Reaktionsgemisch zweimal mit je 150 ml 2N Sodalösung und einmal mit 100 ml Wasser ausgewaschen. Die organische Phase wurde über $MgSO_4$ getrocknet, das Methylenchlorid im Vakuum abdestilliert und der Rückstand 1 Stunde bei 50 °C und 0,065 mbar getrocknet. Ausbeute an (2S,5S)-1-Benzoyl-2-tert.butyl-3-methyl-5-(3'-thiabutyl)-imidazolidin-4-on : 31,5 g (94 % der Theorie). Zur weiteren Reinigung wurde zweimal aus Diethylether umkristallisiert.

Schmelzpunkt : 129 °C
$[\alpha]_D^{25}$ : + 58,8° (c = 1,4 ; $CHCl_3$)
IR ($CHCl_3$) : 2 970 (m), 1 690 (s), 1 650 (s), 1 365 (s), 1 110 (m) $cm^{-1}$
MS : 334 ($M^+$, 1), 278 (100)
$^1$H-NMR ($CDCl_3$) : 7,93-7,30 (m, 5H) <u>H</u>$_{Ar}$ ;
5,66 (s, 1H) C—<u>H</u> ;
4,42 (d, J = 5 Hz, 1H) C<u>H</u>—$(CH_2)_2$—S—$CH_3$
3,06 (s, 3H) C<u>H</u>$_3$—N ;
3,00-1,80 (m, 4H) (C<u>H</u>$_2$)$_2$—S—$CH_3$ ;
1,73 (s, 3H) C<u>H</u>$_3$—S ;
1,06 ppm (s, 9H) (C<u>H</u>$_3$)$_3$—C.

c) Herstellung von (2S, 5R)-1-Benzoyl-2-tert.butyl-3,5-dimethyl-5-(3'-thiabutyl)-imidazolidin-4-on

Zu 3,34 g (10,0 mmol) des nach b) hergestellten (2S,5S)-1-Benzoyl-2-tert.butyl-3-methyl-5-(3'-thiabutyl)-imidazolidin-4-ons, gelöst in 60 ml Tetrahydrofuran, wurden bei — 60 °C 10,6 mmol einer 1M Lösung von Lithiumdiisopropylamid in Tetrahydrofuran zugegeben. Dabei färbte sich die Lösung tiefrot. Nach weiterem 15-minütigen Rühren bei — 60 °C wurden 0,9 ml (15,0 mmol) Methyljodid zugegeben. Das jetzt schwach gelbe Reaktionsgemisch wurde auf Raumtemperatur erwärmen gelassen, in 100 ml etwa halbgesättigter wäßriger $NH_4Cl$-Lösung gegossen und mit insgesamt 200 ml Diethylether extrahiert. Die vereinigten organischen Extrakte wurden mit Wasser gewaschen, über $MgSO_4$ getrocknet und unter

vermindertem Druck vom Lösungsmittel befreit. Der Rückstand wurde aus einem Gemisch von Diethylether und n-Pentan im Volumenverhältnis 1 : 1 umkristallisiert, wonach 2,36 g (66 % der Theorie) an (2S,5R)-1-Benzoyl-2-tert.butyl-3,5-dimethyl-5-(3'-thiabutyl)-imidazolidin-4-on erhalten wurden.

Schmelzpunkt : 105 °C

$[\alpha]_D^{20}$ : — 71,9° (c = 1 ; $CHCl_3$)

$^1H$-NMR ($CDCl_3$) : 7,50 (s, 5H) $\underline{H}_{Ar}$ ;
5,83 (s, 1H) C—$\underline{H}$ ;
3,84 (s, 3H) $C\underline{H}_3$—N ;
3,00-2,00 (m, 4H) $(C\underline{H}_2)_2$ ;
2,03 (s, 3H) S—$C\underline{H}_3$ ;
1,10 ppm (s, 12H) C—$C\underline{H}_3$ und $(C\underline{H}_3)_3$—C.

d) Herstellung von (R)-α-Methylmethionin

1,0 g (2,79 mmol) des nach c) hergestellten (2S,5R)-1-Benzoyl-2-tert.butyl-3,5-dimethyl-5-(3'-thiabutyl)-imidazolidin-4-ons wurden mit 25 ml 20 gewichtsprozentiger wäßriger Salzsäure 4 Stunden im Bombenrohr auf 180 °C erhitzt. Das Hydrolysegemisch wurde filtriert, mit 30 ml Methylenchlorid extrahiert und die wäßrige Phase im Vakuum zur Trockne eingedampft. Der Rückstand wurde in 30 ml Wasser aufgenommen und mittels eines Ionenaustauschers dehydrohalogeniert. Die so erhaltene wäßrige Lösung des freien (R)-α-Methylmethionins wurde eingedampft und der kristalline Rückstand bei 50 °C im Vakuum bis zur Gewichtskonstanz getrocknet. Ausbeute 0,402 g (88 % der Theorie).

$[\alpha]_D^{25}$ = — 17,9° (c = 0,7 ; 0,2N HCl)

$^1H$-NMR ($D_2O$) : 2,83-2,56 (m, 2H) S—$C\underline{H}_2$ ;
2,32-2,03 (m, 2H) C—$C\underline{H}_2$ ;
2,23 (s, 3H) S—$C\underline{H}_3$ ;
1,63 ppm (s, 3H) C—$C\underline{H}_3$.

Referenz (HDO) : 4,90 ppm

## Beispiel 3

a) Herstellung von (S)-N-(2', 2'-Dimethylpropyliden)-alaninmonomethylamid

Zu 30,1 g (295 mmol) (S)-Valinmonomethylamid, gelöst in 100 ml n-Pentan, wurden 33,0 ml (300 mmol) Pivalaldehyd zugegeben. Das Reaktionsgemisch wurde am Wasserabscheider gekocht, bis die Wasserbildung beendet war (3,5 Stunden). Das Lösungsmittel wurde unter vermindertem Druck entfernt und es hinterblieben 44,0 g (88 % der Theorie) an (S)-N-(2',2'-Dimethylpropyliden)-alaninmonomethylamid, das ohne weitere Reinigung weiterverarbeitet wurde. Für die Ermittlung der exakten Stoffdaten wurde eine Probemenge durch Destillation gereinigt.

Siedepunkt : 140 °C/0,065 mbar

$[\alpha]_{365}^{20}$ : + 219,6° (c = 5,3 ; $CHCl_3$)

Elementaranalyse :

$C_9H_{18}N_2O$ (170,23)

Berechnet : %C 63,49 %H 10,66

Gefunden : %C 63,57 %H 10,74

IR ($CHCl_3$) : 3 330 (m), 2 980 (s), 1 665 (s), 1 520 (m), 1 415 (m) $cm^{-1}$

$^1H$-NMR ($CDCl_3$) : 7,57 (s, 1H) $\underline{H}$—C = N ;
6,98 (s̄, 1H) N—$\underline{H}$ ;
3,67 (q, J = 7 Hz, 1H) C—$\underline{H}$ ;
2,84 (d, J = 5 Hz, 3H) $C\underline{H}_3$—N ;
1,30 (d, J = 7 Hz, 3H) $C\underline{H}_3$—C ;
1,06 ppm (s, 9H) $(C\underline{H}_3)_3$—C.

b) Herstellung von (2S,5S)-1-Benzoyl-2-tert.butyl-3,5-dimethyl-imidazolidin-4-on

13,6 g (80 mmol) des nach a) hergestellten (S)-N-(2',2'-Dimethylpropyliden)-alaninmonomethylamids als Lösung in 30 ml Methanol wurden unter Kühlung auf 0 °C mit 60 ml einer gesättigten methanolischen Salzsäure versetzt und 30 Minuten bei 0 °C und anschließend 2 Stunden bei 25 °C gerührt. Das Lösungsmittel wurde bei 25 °C unter vermindertem Druck entfernt und der Rückstand in 100 ml Methylenchlorid aufgenommen. Die Methylenchloridlösung wurde bei 0 °C mit 9,3 ml (80 mmol) Benzoylchlorid und 22,2 ml (160 mmol) Triethylamin versetzt. Nach dem Erwärmen auf 25 °C wurde das Reaktionsgemisch zweimal mit je 150 ml 2N Sodalösung und einmal mit 100 ml Wasser ausgewaschen. Die organische Phase wurde über $MgSO_4$ getrocknet, das Methylenchlorid im Vakuum abdestilliert und der Rückstand 1 Stunde bei 50 °C und 0,065 mbar getrocknet. Ausbeute an (2S,5S)-1-Benzoyl-2-tert.butyl-3,5-dimethyl-imidazolidin-4-on : 21,0 g (96 % der Theorie). Zur weiteren Reinigung wurde zweimal aus Diethylether umkristallisiert.

Schmelzpunkt : 175 °C

$[\alpha]_D^{25}$ : + 44,5° (c = 1,0 ; $CHCl_3$)
IR (KBr) : 2 980 (m), 1 700 (s), 1 365 (s), 1 380 (s), 1 260 (m) $cm^{-1}$
Elementaranalyse :
$C_{16}H_{22}N_2O_2$ (274,34)
Berechnet : %C 70,04 %H 8,08 %N 10,21
Gefunden : %C 69,96 %H 8,16 %N 10,23
$^1$H-NMR ($CDCl_3$) : 7,93-7,30 (m, 5H) $\underline{H}_{Ar}$ ;
5,67 (s, 1H) C$\underline{H}$ ;
4,27 (q, J = 7 Hz, 1H) C$\underline{H}$—$CH_3$ ;
3,04 (s, 3H) C$\underline{H}_3$—N ;
1,05 (s, 9H) $(C\underline{H}_3)_3$—C ;
0,97 ppm (d, J = 7 Hz, 3H) C$\underline{H}_3$—CH.

c) Herstellung von (2S,5S)-1-Benzoyl-2-tert.butyl-5-(3',4'-dimethoxybenzyl)-3,5-dimethyl-imidazolidin-4-on

Zu 2,74 g (10 mmol) des nach b) hergestellten (2S,5S)-1-Benzoyl-2-tert.butyl-3,5-dimethyl-imidazolidin-4-ons, gelöst in 60 ml Tetrahydrofuran, wurden bei —78 °C 11 mmol einer 1M Lösung von n-Butyllithium in Hexan zugegeben. Dabei färbte sich die Lösung tiefrot. Nach 15-minütigem Rühren bei —78 °C wurden 2,8 g (12 mmol) 3,4-Dimethoxybenzylbromid in 15 ml Tetrahydrofuran zugegeben. Das Reaktionsgemisch wurde auf Raumtemperatur erwärmen gelassen. Das jetzt schwach gelbe Reaktionsgemisch wurde in 100 ml etwa halbgesättigter wäßriger $NH_4Cl$-Lösung gegossen und mit insgesamt 200 ml Diethylether extrahiert. Die vereinigten organischen Extrakte wurden mit Wasser gewaschen, über $MgSO_4$ getrocknet und unter vermindertem Druck vom Lösungsmittel befreit. Der Rückstand wurde zur weiteren Reinigung mit einem Gemisch von Diethylether und n-Pentan im Volumenverhältnis 1 : 1 als Laufmittel chromatographiert. Nach dem Abdampfen des Lösungsmittels aus dem Eluat hinterblieben 2,64 g (61 % der Theorie) an (2S,5S)-1-Benzoyl-2-tert.butyl-5-(3',4'-dimethoxybenzyl)-3,5-dimethyl-imidazolidin-4-on.

Schmelzpunkt : 165 °C
$[\alpha]_D^{25}$ : + 74,9° (c = 0,4 ; $CHCl_3$)
IR (KBr) : 2 960 (m), 1 710 (s), 1 640 (s), 1 520 (s), 1 370 (m), 1 260 (m), 1 240 (m), 1 135 (m) $cm^{-1}$
MS : 424 ($M^+$, 05), 105 (100)
$^1$H-NMR ($CDCl_3$) : 7,80-6,67 (m, 8H) $\underline{H}_{Ar}$ ;
5,26 ; 4,92 (s, 1H) C—$\underline{H}$ ;
3,90 ; 3,51 (s, 3H) C$\underline{H}_3$—O ;
3,83 (s, 3H) C$\underline{H}_3$—O ;
3,80-3,00 (m, 2H) C—C$\underline{H}_2$ ;
2,97 ; 2,63 (s, 3H) C$\underline{H}_3$—N ;
2,90 ; 2,60 (s, 3H) C$\underline{H}_3$—C ;
1,00 ; 0,70 ppm (s, 9H) $(C\underline{H}_3)_3$—C.

d) Herstellung von (S)-α-Methyl-N-acetyl-β-(3',4'-diacetoxyphenyl-alanin (Triacetylderivat von (S)-α-Methyldopa)

4,69 g (11,05 mmol) des nach c) hergestellten (2S,5S)-1-Benzoyl-2-tert.butyl-5-(3',4'-dimethoxybenzyl)-3,5-dimethylimidazolidin-4-ons wurden mit 30 ml 20 gewichtsprozentiger wäßriger Salzsäure 4 Stunden im Bombenrohr auf 180 °C erhitzt. Das Hydrolysegemisch wurde filtriert, mit 30 ml Methylenchlorid extrahiert und die wäßrige Phase bei 20 mbar zur Trockne eingedampft. Der Rückstand wurde in 20 ml Pyridin aufgenommen und bei 0,01 mbar erneut zur Trockne eingedampft. Der nun verbliebene Rückstand wurde mit 40 ml Acetanhydrid und 20 ml Pyridin versetzt und 3 Stunden auf 95 °C erhitzt. Nach dem Abkühlen wurde erneut zur Trockne eingedampft. Das hinterbliebene Öl wurde in 10 ml Wasser, 5 ml Aceton und 1 ml 2,5M wäßriger Salzsäure gelöst. Nach erneutem Eindampfen wurde der nun verbleibende Rückstand in 20 ml Ethanol gelöst und zu einer Mischung von 50 ml Wasser und 150 ml Diethylether zugegeben. Die wäßrige Phase wurde noch zweimal mit je 100 ml Diethylether extrahiert. Nach dem Abdampfen des Diethylethers wurde der Rückstand aus Aceton/n-Pentan im Volumenverhältnis 1 : 1 umkristallisiert und es wurden 2,44 g (56 % der Theorie) des Triacetylderivats von (S)-α-Methyldopa erhalten.

Schmelzpunkt : 178-179 °C (Literaturangabe : 180-181 °C)
$[\alpha]_D^{25}$ : —93,9° (c = 1,04 ; $CH_3OH$)
$^1$H-NMR ($CD_3OD$) : 7,20-6,91 (m, 3H) $\underline{H}_{Ar}$ ;
4,85 ($\bar{s}$, 2H) N$\underline{H}$, COO$\underline{H}$ ;
3,46 ; 3,11 (AB, J = 13,5 Hz, 2H) 2 × C—$\underline{H}$ ;
2,21 (s, 6H) 2 × OOC—C$\underline{H}_3$ ;
1,90 (s, 3H) NH—CO—C$\underline{H}_3$ ;
1,40 ppm (s, 3H)—C—C$\underline{H}_3$.

Beispiel 4

a) Herstellung von (S)-N-(2',2'-Dimethylpropyliden)-alaninmonomethylamid

Die Herstellung erfolgte wie im Beispiel 3a).

b) Herstellung von (2R,5S)-1-Benzoyl-2-tert.butyl-3,5-dimethyl-imidazolidin-4-on

13,6 g (80 mmol) des nach a) hergestellten (S)-N-(2',2'-Dimethylpropyliden)-alaninmonomethylamids wurden mit 20,0 g (88 mmol) Benzoesäureanhydrid 3 Stunden lang auf 130 °C erhitzt. Nach dem Abkühlen wurde die erstarrte Masse in 200 ml Methylenchlorid aufgenommen und die Methylenchloridlösung zweimal mit je 150 ml 2N Sodalösung und einmal mit 100 ml Wasser ausgewaschen. Die organische Phase wurde über $MgSO_4$ getrocknet und das Methylenchlorid unter vermindertem Druck abdestilliert. Der Rückstand wurde zur Reinigung zweimal aus einem Gemisch von Diethylether und Methylenchlorid im Volumenverhältnis 1 : 1 umkristallisiert. Ausbeute an (2R,5S)-1-Benzoyl-2-tert.butyl-3,5-dimethyl-imidazolidin-4-on : 20,0 g (91 % der Theorie).

Schmelzpunkt : 114-117 °C
$[\alpha]_D^{25}$ : — 47,7° (c = 1,04 ; $CHCl_3$)
IR (KBr) : 2 980 (m), 1 700 (s), 1 665 (s), 1 630 (m), 1 360 (s) $cm^{-1}$
Elementaranalyse :
$C_{16}H_{22}N_2O_2$ (274,34)
Berechnet : %C 70,04 %H 8,08 %N 10,21
Gefunden : %C 69,99 %H 8,11 %N 10,16
$^1H$-NMR ($CDCl_3$) : 7,40 (s, 5H) $\underline{H}_{Ar}$ ;
5,60 (s, 1H) $C\underline{H}$ ;
3,83 (g, J = 7 Hz, 1H) $C\underline{H}—CH_3$ ;
3,02 (s, 3H) $C\underline{H}_3—N$ ;
2,42 (d, J = 7 Hz, 3H) $C\underline{H}_3—CH$ ;
1,10 ppm (s, 9H) $(C\underline{H}_3)_3—C$.

c) Herstellung von (2R,5R)-1-Benzoyl-2-tert.butyl-5-(3',4'-dimethoxybenzyl)-3,5-dimethyl-imidazolidin-4-on.

Zu 2,74 g (10 mmol) des nach b) hergestellten (2R,5S)-1-Benzoyl-2-tert.butyl-3,5-dimethyl-imidazolidin-4-ons, gelöst in 60 ml Tetrahydrofuran, wurden bei — 78 °C 11 mmol einer 1M Lösung von Lithiumdiisopropylamid in einer Mischung von Tetrahydrofuran und n-Hexan im Volumenverhältnis 1 : 3 zugegeben. Dabei färbte sich die Lösung tiefrot. Nach 30-minütigem Rühren bei — 78 °C wurden 2,8 g (12 mmol) 3,4-Dimethoxybenzylbromid in 15 ml Tetrahydrofuran zugegeben. Das Reaktionsgemisch wurde innerhalb von 2 Stunden auf Raumtemperatur erwärmen gelassen. Das jetzt schwach gelbe Reaktionsgemisch wurde in 100 ml etwa halbgesättigter wäßriger $NH_4Cl$-Lösung gegossen und mit insgesamt 400 ml Diethylether extrahiert. Die vereinigten organischen Extrakte wurden mit Wasser gewaschen, über $MgSO_4$ getrocknet und unter vermindertem Druck vom Lösungsmittel befreit. Der Rückstand wurde zur weiteren Reinigung mit einem Gemisch von Diethyleter und n-Pentan im Volumenverhältnis 5 : 1 als Laufmittel chromatographiert. Nach dem Abdampfen des Lösungsmittels aus dem Eluat hinterblieben 2,47 g (57 % der Theorie) an (2R,5R)-1-Benzoyl-2-tert.butyl-5-(3',4'-dimethoxybenzyl)-3,5-dimethylimidazolidin-4-on.

Schmelzpunkt : 165 °C
$[\alpha]_D^{25}$ : — 78,0° (c = 0,5 ; $CHCl_3$)
IR (KBr) : 2 960 (m), 1 640 (s), 1 520 (m), 1 370 (m), 1 260 (m), 1 240 (m), 1 135 (m) $cm^{-1}$
$^1H$-NMR ($CDCl_3$) : 7,80-6,67 (m, 8H) $\underline{H}_{Ar}$ ;
5,26 ; 4,92 (s, 1H) $C—\underline{H}$ ;
3,90 ; 3,51 (s, 3H) $O—C\underline{H}_3$ ;
3,83 (s, 3H) $O—C\underline{H}_3$ ;
3,80-3,00 (m, 2H) $C—C\underline{H}_2$ ;
2,97-2,60 (s, 3H) $C—C\underline{H}_3$ ;
1,00 ; 0,77 ppm (s, 9H) $—C—(C\underline{H}_3)_3$.

d) Herstellung von (R)-α-Methyl-β-(3',4'-dihydroxyphenyl)-alanin ((R)-α-Methyldopa)

0,6 g (1,41 mmol) des nach c) hergestellten (2R,5R)-1-Benzoyl-2-tert.butyl-5-(3',4'-dimethoxybenzyl)-3,5-dimethylimidazolidin-4-ons wurden mit 20 ml 20 gewichtsprozentiger wäßriger Salzsäure 4 Stunden im Bombenrohr auf 180 °C erhitzt. Das Hydrolysegemisch wurde filtriert, mit 15 ml Methylenchlorid extrahiert und die wäßrige Phase bei 20 mbar zur Trockne eingedampft. Es hinterblieben 0,47 g des Methylammoniumsalzes von (R)-α-Methyldopa.

$^1H$-NMR ($D_2O$) : 6,91-6,50 (m, 3H) $\underline{H}_{Ar}$ ;

3,10 ; 2,86 (AB, J = 13,5 Hz, 2H) 2 × C—H ;
2,53 (s, 3H) $CH_3$—N ;
1,55 ppm (s, 3H) C—$CH_3$.

**Patentansprüche**

1. Verfahren zur enantioselektiven Herstellung von α-alkyllierten, acyclischen α-Aminocarbonsäuren der allgemeinen Formel

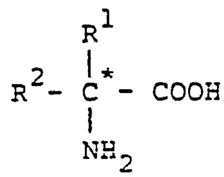

(I)

in der* ein Asymmetriezentrum, $R^1$ eine Methyl-, Ethyl-, n-Propyl-, n-Butyl-, Allyl-, Benzyl- oder substituierte Benzylgruppe und $R^2$ eine Methyl-, Ethyl-, n-Propyl-, i-Propyl-, n-Butyl-, sekundäre Butyl-, i-Butyl-, Methoxymethyl-, Methylmercaptomethyl-, 2-Methylmercaptoethyl-, 2-Ethylmercaptoethyl-, Phenyl-, Benzyl- oder eine in beliebiger Ringstellung 1- bis 3-fach durch eine Alkyl- oder Alkoxygruppe oder durch Fluor oder Chlor substituierte Benzylgruppe bedeuten, dadurch gekennzeichnet, daß man ein Imidazolidin-4-on der allgemeinen Formel

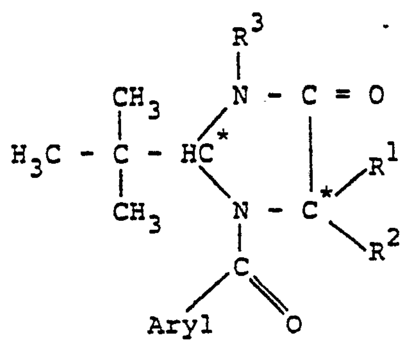

(II)

in der *, $R^1$ und $R^2$ die bereits angegebene Bedeutung haben und $R^3$ eine Methyl- oder Ethylgruppe und Aryl eine Phenyl- oder substituierte Phenylgruppe bedeuten, sauer verseift.

2. Verfahren nach Anspruch 1, dadurch gekennzeichnet, daß man das Imidazolidin-4-on der allgemeinen Formel (II) durch Alkylierung eines Enolats der allgemeinen Formel

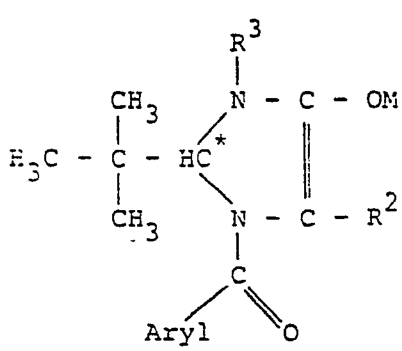

(III)

in der*, $R^2$, $R^3$ und Aryl die bereits angegebene Bedeutung haben und M für Lithium, Natrium oder Kalium steht, mit einem Alkylierungsmittel der allgemeinen Formel

$$R^1—X \qquad (IV),$$

in der $R^1$ die bereits angegebene Bedeutung hat und X eine Austrittsgruppe aus der Reihe Chlorid, Bromid, Jodid, Tosylat, Mesylat oder Trifluormethylsulfonat bedeutet, herstellt.

3. Verfahren nach Anspruch 1 oder 2, dadurch gekennzeichnet, daß man das Enolat der allgemeinen Formel (III) durch Umsetzung eines Imidazolidin-4-ons der allgemeinen Formel

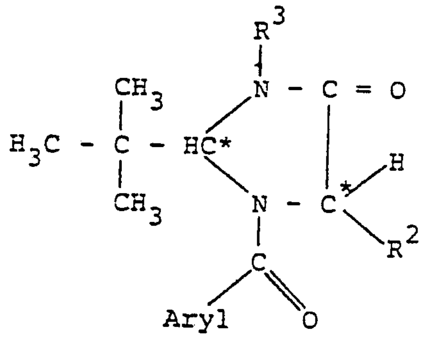

(V)

in der *, $R^2$, $R^3$ und Aryl die bereits angegebene Bedeutung haben, mit einer starken Base der allgemeinen Formel

M—Y (VI),

in der M die bereits angegebene Bedeutung hat und Y Wasserstoff oder eine n-Butyl-, tert.-Butylat-, Amino-, Dimethylamino-, Diethylamino-, Di-n-propylamino, Di-isopropylamino-, Di-(trimethylsilyl)-amino- oder Phenylgruppe bedeutet, herstellt.

4. Verfahren nach einem der Ansprüche 1 bis 3, dadurch gekennzeichnet, daß man das Imidazolidin-4-on der allgemeneinen Formel (V) durch Cyclisierung eines Imins (Schiff'sche Base) der allgemeinen Formel

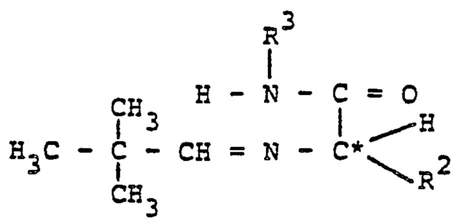

(VII)

in der *, $R^2$ und $R^3$ die bereits angegebene Bedeutung haben, unter gleichzeitiger oder anschließender Einführung der Gruppe

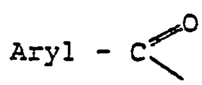

herstellt.

5. Verfahren nach einem der Ansprüche 1 bis 4, dadurch gekennzeichnet, daß man das Imin der allgemeinen Formel (VII) durch Umsetzung eines α-Aminocarbonsäureamids der allgemeinen Formel

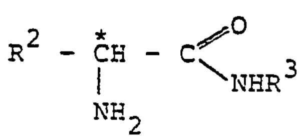

(VIII)

in der *, $R^2$ und $R^3$ die bereits angegebene Bedeutung haben, mit Pivalaldehyd herstellt.

## Claims

1. A process for the enantio-selective production of α-alkylated acyclic α-aminocarboxylic acids corresponding to the general formula

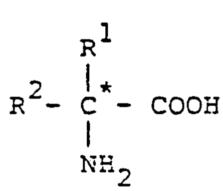

(I)

in which * represents a centre of asymmetry, $R^1$ represents a methyl, ethyl, n-propyl, n-butyl, allyl, benzyl or substituted benzyl group and $R^2$ represents a methyl, ethyl, n-propyl, i-propyl, n-butyl, secondary butyl, i-butyl, methoxymethyl, methylmercaptomethyl, 2-methylmercaptoethyl, 2-ethylmercaptoethyl, phenyl, benzyl group or a benzyl group substituted once to three times in an optional ring position by an alkyl or alkoxy group or by fluorine or chlorine, characterised in that an imidazolidin-4-one corresponding to the general formula

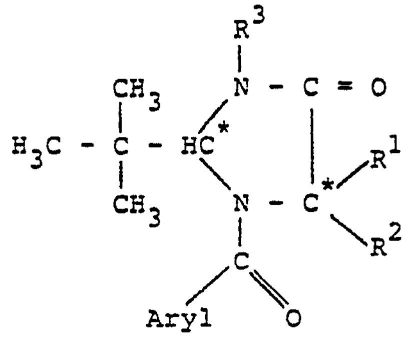

(II)

in which *, $R^1$ and $R^2$ have the meaning already given and $R^3$ represents a methyl or ethyl group and aryl represents a phenyl or substituted phenyl group, is acidly saponified.

2. A process according to claim 1, characterised in that the imidazolidin-4-one corresponding to general formula (II) is produced by alkylation of an enolate corresponding to the general formula

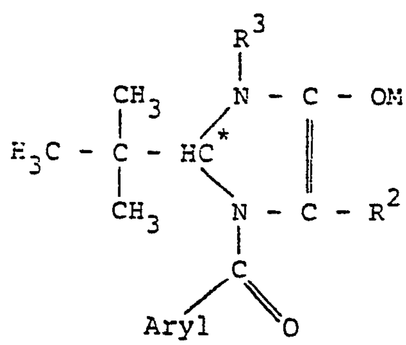

(III)

in which *, $R^2$, $R^3$ and aryl have the meaning given above and M represents lithium, sodium or potassium, with an alkylation agent corresponding to the general formula

$$R^1—X \quad (IV),$$

in which $R^1$ has the meaning given above and X represents an elimination group from the chloride, bromide, iodide, tosylate, mesylate or trifluoromethylsulphonate series.

3. A process according to claim 1 or 2, characterised in that the enolate corresponding to the general formula (III) is produced by reaction of an imidazolidin-4-one corresponding to the general formula

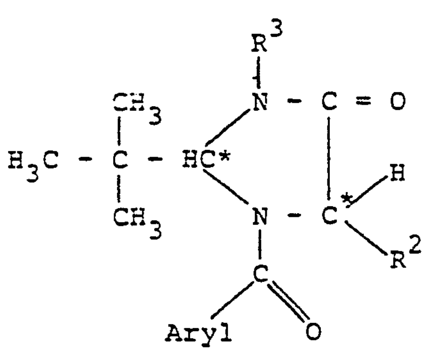

(V)

in which *, $R^2$, $R^3$ and aryl have the meaning given above with a strong base corresponding to the general formula

$$M—Y \quad (VI),$$

in which M has the meaning given above and Y represents hydrogen or an n-butyl, tert-butylate, amino, dimethylamino, diethylamino, di-n-propylamino, di-iso-propylamino, di-(trimethylsilyl)amino or phenyl group.

4. A process according to one of claims 1 to 3, characterised in that the imidazolidin-4-one corresponding to the general formula (V) is produced by cyclization of an imine (Schiff base) corresponding to the general formula

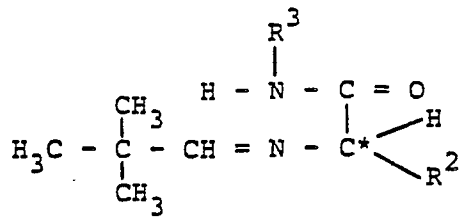 (VII)

in which *, $R^2$ and $R^3$ have the meaning given above with simultaneous or subsequent introduction of the group

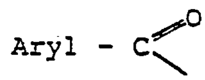

5. A process according to one of claims 1 to 4, characterised in that the imine corresponding to the general formula (VII) is produced by reaction of an α-aminocarboxylic acid amide, corresponding to the general formula

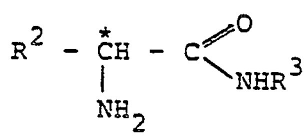 (VIII)

in which *, $R^2$ and $R^3$ have the meaning given above with pivalaldehyde.

**Revendications**

1. Procédé pour la fabrication énantiosélective d'acides α-aminocarboxyliques acycliques, alcoylés en α, répondant à la formule générale

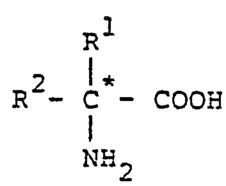 (I)

où * est un centre d'asymétrie, $R^1$ est un groupe méthyl, éthyl, n-propyl, n-butyl, allyl, benzyl ou benzyl substitué, et $R^2$ représente un groupe méthyl, éthyl, n-propyl, i-propyl, n-butyl, butyl secondaire, i-butyl, méthoxyméthyl, méthylmercaptométhyl, 2-méthylmercaptoéthyl, 2-éthylmercaptoéthyl, phényl, benzyl, un groupe benzyl substitué, dans une position quelconque du cycle, 1 à 3 fois par un groupe alcoyl ou alcoxy, ou par un fluor, ou un chlore, qui est caractérisé en ce que l'on fait subir une saponification acide à une imidazolidine-4-one, répondant à la formule générale

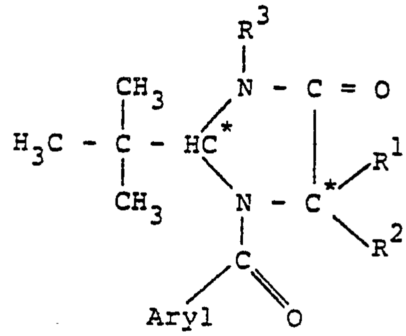 (II)

où *, $R^1$ et $R^2$, ont la signification déjà indiquée, et $R^3$ représente un groupe méthyl ou éthyl, et aryl un groupe phényl ou phényl substitué.

2. Procédé suivant la revendication 1, caractérisé en ce qu'on fabrique l'imidazolidine-4-one de formule générale (II) par alcoylation d'un énolate de formule générale

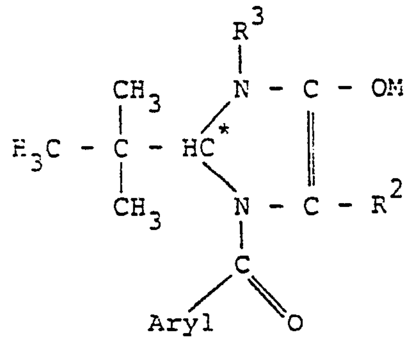

(III)

dans laquelle *, $R^2$, $R^3$ et aryl ont la signification déjà indiquée et M représente lithium, sodium, ou potassium, avec un agent d'alcoylation de formule générale :

$$R^1—X \quad (IV),$$

dans laquelle $R^1$ représente un groupe méthyl, éthyl, n-propyl, n-butyl, allyl, benzyl ou benzyl substitué, et X un groupe de sortie de la série chlorure, bromure, iodure, tosylate, mésylate ou trifluorméthylsulfonate.

3. Procédé suivant les revendications 1 ou 2, caractérisé en ce que l'on fabrique l'énolate de formule générale (III) par réaction d'une imidazolidine-4-one de formule générale

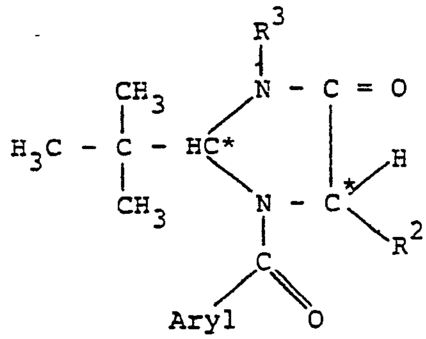

(V)

où *, $R^2$, $R^3$ et aryl ont la signification déjà indiquée, avec une base forte de formule générale :

$$M—Y \quad (VI),$$

où M a la signification déjà indiquée et Y est un hydrogène ou un groupe n-butyl, butylate tert., amino, diméthylamino, diéthylamino, di-n-propylamino, di-isopropylamino, di-(triméthylsilyl)-amino ou phényl.

4. Procédé suivant l'une des revendications 1 à 3, caractérisé en ce qu'on fabrique l'imidazoline-4-one de formule générale (V) par cyclisation d'une imine (base de Schiff) de formule générale :

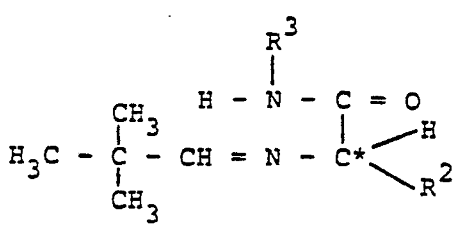

(VII)

où *, $R^2$ et $R^3$ ont la signification déjà indiquée, avec introduction simultanée ou subséquente du groupe :

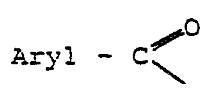

5. Procédé suivant l'une des revendications 1 à 4, caractérisé en ce que l'on fabrique l'imine de formule générale (VII) par réaction d'un amide de l'acide α-aminocarboxylique de formule générale :

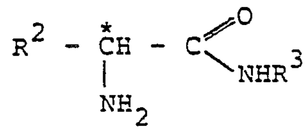 (VIII)

où *, $R^2$ et $R^3$, ont la signification déjà indiquée, avec l'aldéhyde pivalique.